# EUROPÄISCHE PATENTSCHRIFT

(11) **EP 3 503 349 B1**
(45) Veröffentlichungstag und Bekanntmachung des Hinweises auf die Patenterteilung: **15.07.2020**
(21) Anmeldenummer: 17210291.5
(22) Anmeldetag: 22.12.2017
(51) Int. Cl.: H02J 50/10, A61B 6/00, H01F 38/14, H04B 5/00

(54) **DATENÜBERTRAGUNGSEINHEIT UND BILDGEBUNGSVORRICHTUNG MIT EINER ENTSPRECHENDEN DATENÜBERTRAGUNGSEINHEIT**
DATA TRANSMISSION UNIT AND IMAGING APPARATUS COMPRISING A CORRESPONDING DATA TRANSMISSION UNIT
APPAREIL DE TRANSMISSION DE DONNEES ET APPARAEIL D'IMAGERIE MEDICALE COMPRENANT UN TEL APPAREIL DE TRANSMISSION DE DONNEES

(43) Veröffentlichungstag der Anmeldung: 26.06.2019
(73) Patentinhaber: Siemens Healthcare GmbH, 91052 Erlangen (DE)
(72) Erfinder: Fackelmeier, Andreas, 91177 Thalmässing (DE)

(56) Entgegenhaltungen:
- DE-A1- 10 206 160
- DE-A1-102013 001 667
- DE-A1-102014 206 295
- US-A- 5 787 212

## Beschreibung

Die Erfindung betrifft eine Datenübertragungseinheit aufweisend eine Sendeeinheit sowie eine relativ zur Sendeeinheit bewegbare Empfangseinheit und eingerichtet zur Übertragung von Daten von der Sendeeinheit zur Empfangseinheit über einen Koppler auch während einer Relativbewegung zwischen Sendeeinheit und Empfangseinheit, wobei der Koppler einen ersten dielektrischen Wellenleiter aufweist. Zudem betrifft die Erfindung eine Bildgebungsvorrichtung mit einer entsprechenden Datenübertragungseinheit.

Ein Computertomograph weist typischerweise eine feststehende Basis und eine sogenannte Gantry auf, wobei sich die Gantry im Betrieb, also während einer Datenerfassung mittels eines Röntgendetektors, relativ zur feststehenden Basis bewegt und hierbei eine Rotationsbewegung ausführt. Dabei ist der Röntgendetektor, mit dessen Hilfe die Generierung von Daten für eine Bilderzeugung erfolgt, üblicherweise Teil der Gantry, so dass die in Betrieb generierten Daten von der Gantry über eine Datenübertragungsstrecke hin zur feststehenden Basis und üblicherweise hin zu einer Aufbereitungseinheit für die generierten Daten übertragen werden müssen.

Die Übertragung der generierten Daten über die Datenübertragungsstrecke erfolgt dabei typischerweise ohne eine Zwischenspeicherung oder relevante Pufferung und dementsprechend quasi parallel zur Generierung von entsprechenden Daten und somit also auch während der Rotation der Gantry. Hierfür geeignete Datenübertragungsstrecken sind prinzipiell aus dem Stand der Technik bekannt, wobei in den meisten Fällen ein sogenannter Schleifring Teil einer entsprechenden Datenübertragungsstrecke ist.

DE 10 2014 206 295 A1 beschreibt einen Wellenleiter mit Haltestruktur, der bei Bildgebungsvorrichtungen eingesetzt wird.

Eine alternative Ausführung einer für eine derartige Datenübertragung geeigneten Datenübertragungsstrecke ist in der DE 10 2016 208 539 A1 beschrieben. Diese umfasst einen dielektrischen Wellenleiter und ist auch für eine kontaktlose Datenübertragung geeignet. Ausgehend hiervon liegt der Erfindung die Aufgabe zugrunde, eine vorteilhafte Lösung zur Datenübertragung anzugeben.

Diese Aufgabe wird erfindungsgemäß gelöst durch eine Datenübertragungseinheit mit den Merkmalen des Anspruchs 1 sowie durch eine Bildgebungsvorrichtung mit den Merkmalen des Anspruchs 10. Bevorzugte Weiterbildungen sind in den rückbezogenen Ansprüchen enthalten. Die im Hinblick auf die Datenübertragungseinheit angeführten Vorteile und bevorzugten Ausgestaltungen sind sinngemäß auch auf die Bildgebungsvorrichtung übertragbar und umgekehrt.

Eine entsprechende Datenübertragungseinheit weist dabei eine Sendeeinheit sowie eine relativ zur Sendeeinheit bewegbare Empfangseinheit auf und ist eingerichtet zur Übertragung, insbesondere zur kontaktlosen Übertragung, von Daten von der Sendeeinheit zur Empfangseinheit über einen Koppler, und zwar zweckdienlicherweise auch während einer Relativbewegung zwischen der Sendeeinheit und der Empfangseinheit. Hierbei weist der Koppler weiter einen ersten dielektrischen Wellenleiter auf, der seinerseits einen Leiterrumpf und eine Haltestruktur aufweist.

Dabei dient der Leiterrumpf vor allem zur Durchleitung von elektromagnetischen Wellen, wohingegen die Haltestruktur in erster Linie dazu genutzt wird, den ersten dielektrischen Wellenleiter und insbesondere den Leiterrumpf an einer dafür vorgesehenen Baugruppe oder Baueinheit zu fixieren und somit den Leiterrumpf an der entsprechenden Baugruppe bzw. Baueinheit zu halten. Das bedeutet, dass der erste dielektrische Wellenleiter bevorzugt derart ausgestaltet und/oder derart an einer dafür vorgesehenen Baugruppe oder Baueinheit fixiert ist, dass sich die elektromagnetischen Felder bei einer Durchleitung von elektromagnetischen Wellen durch den ersten dielektrischen Wellenleiter im Wesentlichen nur über das Volumen des Leiterrumpfes hinweg ausdehnen und insbesondere nur in geringem Ausmaße in das Volumen der Haltestruktur hinein ausdehnen.

Weiter wird der erste dielektrische Wellenleiter üblicherweise durch einen zweiten dielektrischen Wellenleiter ergänzt, wobei der erste dielektrische Wellenleiter typischerweise Teil der Sendeeinheit und der zweite dielektrische Wellenleiter zweckdienlicherweise Teil der Empfangseinheit ist. Im Betrieb der Datenübertragungseinheit erfolgt dann üblicherweise eine Datenübertragung, insbesondere eine kontaktlose Datenübertragung, vom ersten dielektrischen Wellenleiter zum zweiten dielektrischen Wellenleiter beispielsweise über einen die beiden dielektrischen Wellenleiter trennenden Luftspalt hinweg und dementsprechend sind die beiden dielektrischen Wellenleiter typischerweise auch Teil des Kopplers.

Eine entsprechende Datenübertragung mittels dielektrischen Wellenleitern erfolgt dabei zweckdienlicherweise nach an sich bekanntem Prinzip. Je nach Ausführungsvariante ist die Datenübertragungseinheit dabei als monodirektionale oder als bidirektionale Datenübertragungseinheit ausgebildet, wobei insbesondere im letzteren Fall bevorzugt mehrere Koppler der zuvor genannten Art realisiert sind und wobei dann jeder Koppler zumindest zwei dielektrische Wellenleiter aufweist.

Eine derartige Datenübertragungseinheit ist dabei vorzugsweise für eine Bildgebungsvorrichtung, wie beispielsweise ein Computertomograph oder ein Magnetresonanztomograph, ausgebildet und dementsprechend bevorzugt in seiner solchen Bildgebungsvorrichtung realisiert oder verbaut. Das heißt, dass eine hier vorgestellte Datenübertragungseinheit und insbesondere ein nachfolgend näher beschriebener erster dielektrischer Wellenleiter bevorzugt in einer Bildgebungsvorrichtung zum Einsatz kommt, wie sie insbesondere in der DE 10 2016 208 539 A1 oder in der DE 10 2015 223 068 A1 beschrieben ist. Dabei ersetzt dann die hier beschriebene Datenübertragungseinheit die jeweils in diesen Druckschriften beschriebene Datenübertragungseinheiten oder Übertragungsstrecken und insbesondere wird ein dort beschriebener dielektrischer Wellenleiter oder die dort beschriebenen dielektrischen Wellenleiter durch den nachfolgend näher beschriebenen ersten dielektrischen Wellenleiter ersetzt. Es wird daher an dieser Stelle ausdrücklich auf die Beschreibungen in beiden Druckschriften Bezug genommen.

Bevorzug ist hierbei eine Ausführung der Datenübertragungseinheit und insbesondere des ersten dielektrischen Wellenleiters und/oder des zweiten dielektrischen Wellenleiters, bei der der Leiterrumpf des ersten dielektrischen Wellenleiters und/oder der Leiterrumpf des zweiten dielektrischen Wellenleiters eine Art Profilleiste oder eine Art Profilstrang ausbildet und hierbei insbesondere einen durchgängig einheitlichen Querschnitt aufweist.

Der entsprechende Querschnitt ist dabei beispielsweise rund oder quaderförmig ausgestaltet und die Profilleiste bzw. das Strangprofil bildet zweckdienlicherweise ein in eine Längsrichtung ausgedehntes Strangprofil bzw. eine in eine Längsrichtung ausgedehnte Profilleiste aus. Zumindest im verbauten Zustand bildet die Profilleiste bzw. das Strangprofil weiter bevorzugt eine Art Ringkörper aus, der beispielsweise um den Umfang eines zylinderförmigen Körpers herumlaufend angeordnet ist. Ist die Datenübertragungseinheit dann zum Beispiel Teil eines Computertomographen mit einer zylinderförmigen Gantry, so verläuft der erste dielektrische Wellenleiter gemäß einer Ausführungsvariante umfangsseitig um die Gantry herum, ähnlich einem Schleifring bei Computertomographen mit einem solchen.

Weiter ist es von Vorteil, wenn der Leiterrumpf und die Haltestruktur und insbesondere der gesamte erste dielektrische Wellenleiter aus demselben Material bestehen. In einem solchen Fall ist der erste dielektrische Wellenleiter dann insbesondere einteilig, einstückig und somit quasi monolithisch ausgestaltet. Gleiches gilt bevorzugt auch oder alternativ für den zweiten dielektrischen Wellenleiter.

Einer alternativen Ausgestaltung entsprechend ist die Haltestruktur des ersten dielektrischen Wellenleiters und/oder die Haltestruktur des zweiten dielektrischen Wellenleiters als eine Art separate Struktur ausgebildet, wobei in diesem Fall die Haltestruktur und der Leiterrumpf typischerweise aus unterschiedlichen Materialen bestehen. Hierbei ist der Leiterrumpf dann beispielsweise in die Haltestruktur eingebettet und wird auf diese Weise dann von der Haltestruktur gehalten, die ihrerseits wiederum an einer Baugruppe oder Baueinheit fixiert oder verankert ist. Gemäß einer Ausführungsvariante ist die Haltestruktur dann nach Art einer Teilummantelung oder Teil-Umschäumung ausgebildet, bei der der Leiterrumpf im Querschnitt gesehen zumindest in einem Umfangsbereich, also quasi einseitig, freiliegt und in den übrigen Umfangsbereichen von der Haltestruktur umgeben ist.

Sind der Leiterrumpf und die Haltestruktur aus unterschiedlichen Materialien realisiert, so besteht die Haltestruktur bevorzugt aus einem (mikro)porösen oder schaumartigen Material. Günstig ist hierbei zudem ein Material für die Haltestruktur, dessen Dielektrizitätszahl, Permittivitätszahl oder relative Permittivität näherungsweise den Wert 1 aufweist, also zum Beispiel im Bereich 1 bis 1,5 liegt.

Der Leiterrumpf ist des Weiteren bevorzugt aus Teflon oder einem Polyethylen gefertigt und zweckdienlicherweise für die Durchleitung elektromagnetischer Wellen aus dem Frequenzbereich 40 bis 300 GHz, also zum Beispiel etwa 60 GHz, ausgelegt. Selbiges gilt auch für den Leiterrumpf, sofern der Leiterrumpf und die Haltestruktur aus demselben Material gefertigt sind, und darüber hinaus bevorzugt für den gesamten erste dielektrische Wellenleiter und/oder den gesamten zweiten dielektrischen Wellenleiter, sofern dieser bzw. diese aus einem einzigen Material gefertigt sind, also quasi monolithisch ausgestaltet sind.

Insbesondere, wenn der Leiterrumpf und die Haltestruktur und insbesondere der gesamte erste dielektrische Wellenleiter aus demselben Material bestehen, bildet die Haltestruktur weiter bevorzugt einen ersten Teil einer Steckverbindung, also einer mechanischen Steckverbindung, aus, so dass sich der erste dielektrische Wellenleiter beispielsweise einfach durch Einstecken oder Einclipsen befestigen lässt. Das heißt, dass der erste dielektrische Wellenleiter, das Prinzip ist dabei auch auf den zweiten dielektrischen Wellenleiter übertragbar, bevorzugt im Zuge der Realisierung der Datenübertragungseinheit und insbesondere im Zuge der Herstellung einer Bildgebungsvorrichtung mit Hilfe der Haltestruktur und durch Ausbildung einer Steckverbindung an einer Baugruppe oder Baueinheit fixiert wird und somit lösbar an der entsprechenden Baugruppe oder Baueinheit befestigt wird. Dementsprechend weist dann die Baueinheit oder Baugruppe den zweiten Teil der Steckverbindung auf, der beispielsweise durch eine Anzahl Vertiefungen oder Nuten ausgebildet ist.

In vorteilhafter Weiterbildung ist der zweite Teil der Steckverbindung als eine Art Aufnahme mit einer Hinterschneidung ausgebildet. Ist die Steckverbindung dann ausgebildet und liegt die Haltestruktur oder der erste elektrische Wellenleiter in der Aufnahme ein, so hintergreift die Haltestruktur oder der erste dielektrische Wellenleiter quasi die Hinterschneidung. Das heißt, dass die Steckverbindung bevorzugt derart ausgebildet ist, dass die Haltestruktur oder der erste dielektrische Wellenleiter bei ausgebildeter Steckverbindung nicht oder nicht nur durch Kraftschluss sondern bzw. sondern auch durch Formschluss gehalten wird. Für eine einfachere Ausbildung der Steckverbindung ist die Haltestruktur oder der erste dielektrische Wellenleiter dann weiter bevorzugt zumindest teilweise elastisch ausgebildet.

Einer Ausführungsvariante entsprechend weist die Haltestruktur weiter eine Anzahl Haltearme aus oder ist durch eine Anzahl Haltearme ausgebildet, die an den Leiterrumpf angeformt sind und sich typischerweise in Steckrichtung der Steckverbindung erstrecken. Dabei sind die Haltearme bevorzugt gleichmäßig, also insbesondere nach Art einer Gleichteilung, über die Längsausdehnung und insbesondere über die Ausdehnung in Längsrichtung des ersten dielektrischen Wellenleiters verteilt angeordnet.

Alternativ oder ergänzend hierzu bildet der erste dielektrische Wellenleiter eine Art Profilleiste oder Strangprofil aus, welche bzw. welches weiter bevorzugt einen durchgängig einheitlichen Querschnitt aufweist. In diesem Fall ist die Steckverbindung dann ähnlich einer Nut-Feder-Verbindung, ähnlich einer Spundung oder ähnlich einer Schwalbenschwanzverbindung ausgestaltet. Jene Verbindung erstreckt sich gemäß einer Ausführungsvariante dabei bevorzugt über wenigstens 50 % der Längsausdehnung, weiter bevorzugt über wenigstens 75 % und insbesondere im Wesentlichen entlang der gesamten Längsausdehnung des ersten dielektrischen Wellenleiters.

Weiter weist die den ersten dielektrischen Wellenleiter ausbildende Profilleiste vorzugsweise im Querschnitt gesehen eine Anzahl Haltestege auf, die sich quer zur Längsausdehnung, also typischerweise quer zur Ausdehnung in Längsrichtung, erstrecken und zudem bevorzugt auch quer zur Steckrichtung der Steckverbindung, also zu der Richtung, in die der erste dielektrische Wellenleiter zu führen ist, um die Steckverbindung herzustellen. Diese Haltestege bilden gemäß einer weitere bevorzugten Ausführungsvariante die Haltestruktur aus und hintergreifen beispielsweise bei ausgebildeter Steckverbindung eine Hinterschneidung, so dass dann die Steckverbindung nicht oder nicht nur als kraftschlüssige Verbindung ausgebildet ist sondern bzw. sondern auch als formschlüssige Verbindung.

In vorteilhafter Weiterbildung ist im Querschnitt gesehen an zwei gegenüberliegenden Seiten des Leiterrumpfes je ein Haltesteg an den Leiterrumpf angeformt, und zwar insbesondere unmittelbar an den Leiterrumpf ohne eine weitere zwischenliegende geometrische Form. Der erste dielektrische Wellenleiter weist dann im Querschnitt gesehen typischerweise eine Form auf mit einem beispielsweise quaderförmigen Bauch, dem Leiterrumpf, an den sich zwei einander gegenüberliegende Flügel, die beiden Haltestege, anschließen.

Dabei variiert die Form der Haltestege, die Form des Leiterrumpfes und die relative Position, an der die Haltestege am Leiterrumpf angeformt sind, je nach Ausführungsvariante und Anwendungszweck. So verjüngen sich die Haltestege gemäß einer Ausführungsvariante im Querschnitt gesehen nach außen hin, also in eine Richtung quer zur Längsrichtung und quer zur Steckrichtung der Steckverbindung sowie vom Leiterrumpf weggerichtet oder wegzeigend.

Alternativ oder ergänzend hierzu sind die Haltestege im Querschnitt gesehen abgewinkelt, und zwar insbesondere entgegen der Steckrichtung der Steckverbindung. In diesem Fall hintergreifen die Haltestege dann beispielsweise bei ausgebildeter Steckverbindung eine Hinterschneidung nach Art von Widerhaken.

Günstig ist weiter eine Ausführung, bei der sich die Haltestege im Querschnitt gesehen, bündig an eine Oberseite des Leiterrumpfes anschließen, üblicherweise an eine Oberseite, deren Normale entgegen der Steckrichtung der Steckverbindung gerichtet ist. Bei einer solchen Ausführung weist der erste dielektrische Wellenleiter dann im Querschnitt gesehen typischerweise eine Art T-Form auf.

Weiter bevorzugt ist der Kontakt zwischen dem ersten dielektrischen Wellenleiter und der Baueinheit oder Baugruppe, die den zweiten Teil der Steckverbindung aufweist, auf ein Minimum beschränkt, und zwar insbesondere auf den Bereich der äußeren Enden der Haltestege. In allen übrigen Bereichen ist um den ersten dielektrischen Wellenleiter herum bevorzugt ein Freiraum gegeben, in dem typischerweise Luft vorliegt. Dies wirkt sich günstig auf die Dämpfungscharakteristik des ersten dielektrischen Wellenleiters aus.

Wie bereits zuvor erwähnt, ist eine hier vorgestellte Datenübertragungseinheit für eine Bildgebungsvorrichtung ausgebildet und dementsprechend wird eine hier vorgestellte Datenübertragungseinheit bevorzugt in einer Bildgebungsvorrichtung eingesetzt. Eine erfindungsgemäße Bildgebungsvorrichtung ist dann bevorzugt als Computertomograph oder Magnetresonanztomograph ausgebildet und weist eine Datenüberragungseinheit entsprechend einer der zuvor genannten Ausführungsvarianten auf.

Einer bevorzugten Ausführungsvariante entsprechend ist die Bildgebungsvorrichtung dabei als Computertomograph ausgebildet und weist eine feststehende Basis sowie eine rotierbare Gantry mit einer Gantry-Tragstruktur auf. Dabei ist dann die Sendeeinheit bevorzugt Teil der rotierbaren Gantry und der erste dielektrische Wellenleiter, der üblicherweise die Sendeeinheit mit ausbildet, ist bevorzugt an der Gantry-Tragstruktur fixiert. Hierbei ist der erste dielektrische Wellenleiter bevorzugt mit Hilfe einer Steckverbindung, insbesondere einer Steckverbindung der zuvor beschriebenen Art, an der Gantry-Tragstruktur fixiert und in diesem Fall weist die Gantry-Tragstruktur vorzugsweise eine Nut auf, die einen Teil der Steckverbindung ausbildet, insbesondere den zweiten Teil zu einem zuvor beschriebenen ersten Teil, also einem durch den ersten dielektrischen Wellenleiter ausgebildeten ersten Teil.

Eine entsprechende Nut ist dabei weiter bevorzugt als umlaufende Nut ausgebildet, die um den Umfang der Gantry herum verläuft. Außerdem ist es vorteilhaft, wenn der erste dielektrische Wellenleiter nach Ausbildung der Steckverbindung versenkt in der Nut angeordnet ist.

Ausführungsbeispiele der Erfindung werden nachfolgend anhand einer schematischen Zeichnung näher erläutert. Darin zeigen:
- FIG 1: in einer Art Schnittdarstellung ein Computertomograph mit einer Nut und einem in die Nut eingesteckten ersten dielektrischen Wellenleiter,
- FIG 2: in einer Art Schnittdarstellung die Nut und der in die Nut eingesteckte erste dielektrische Wellenleiter,
- FIG 3: in einer Art Schnittdarstellung der erste dielektrische Wellenleiter,
- FIG 4: in einer Art Schnittdarstellung eine erste alternative Ausführung des ersten dielektrischen Wellenleiters,
- FIG 5: in einer Art Schnittdarstellung eine zweite alternative Ausführung des ersten dielektrischen Wellenleiters,
- FIG 6: in einer Art Schnittdarstellung eine dritte alternative Ausführung des ersten dielektrischen Wellenleiters und
- FIG 7: in einer Art Schnittdarstellung eine vierte alternative Ausführung des ersten dielektrischen Wellenleiters.

Einander entsprechende Teile sind in allen Figuren jeweils mit den gleichen Bezugszeichen versehen.

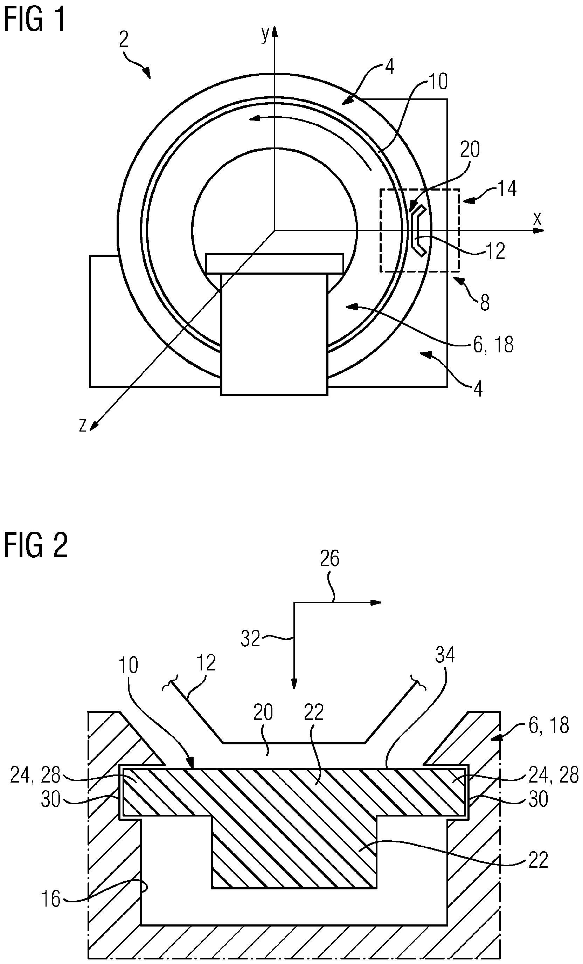

Eine nachfolgend exemplarisch beschriebene und in FIG 1 skizzierte Bildgebungsvorrichtung ist als Computertomograph 2 ausgebildet und weist eine feststehende Basis 4 sowie eine rotierbare Gantry 6 auf. Hierbei ist die Gantry 6 nach an sich bekanntem Prinzip zur Generierung von Bilddaten mittels eines nicht mit abgebildeten Röntgendetektors ausgebildet und dementsprechend werden dann im Betrieb des Computertomographen 2 Daten generiert, die während der Rotation der Gantry 6 von der rotierbaren Gantry 6 zur feststehenden Basis 4 zu übertragen sind und mittels einer Datenübertragungseinheit 8 übertragen werden.

Teil dieser Datenübertragungseinheit 8 sind ein erster dielektrischer Wellenleiter 10 sowie ein zweiter dielektrischer Wellenleiter 12, die derart zueinander angeordnet sind, dass zwischen diesen ein Luftspalt 20 von im Mittel etwa 1 mm gegeben ist, der bei Rotation der Gantry 6 in einem Bereich zwischen 0,5 mm und 1,5 mm variiert. Über diesen Luftspalt 20 hinweg lassen sich nach an sich bekanntem Prinzip Bilddaten vom ersten dielektrischen Wellenleiter 10 zum zweiten dielektrischen Wellenleiter 12 übertragen und somit von der rotierbaren Gantry 6 zur feststehenden Basis 4. Der erste dielektrische Wellenleiter 10 ist somit Teil einer Sendeeinheit, während der zweite dielektrische Wellenleiter 12 Teil einer Empfangseinheit ist. Zudem bilden der erste dielektrische Wellenleiter 10 und der zweite dielektrische Wellenleiter 12 einen Koppler 14 mit aus, der die Basis 4 und die Gantry 6 signaltechnisch koppelt.

In bevorzugter Ausgestaltung ist der erste dielektrische Wellenleiter 10 dabei ringförmig gestaltet oder nimmt im montierten Zustand eine ringförmige Gestalt an, wie dies in FIG 1 angedeutet ist. Der erste dielektrische Wellenleiter 10 verläuft somit im montierten Zustand um den Umfang der rotierbaren Gantry 6 herum und ist dabei bevorzugt in einer Nut 16 versenkt angeordnet. Jene Nut 16 ist hierbei Teil einer Gantry-Tragstruktur 18 und bildet bevorzugt einen zweiten Teil einer Steckverbindung aus, mittels derer der erste dielektrische Wellenleiter 10 an der Gantry-Tragstruktur 18 befestigt ist.

Den zweiten Teil der Steckverbindung ergänzenden ersten Teil der Steckverbindung bildet im Ausführungsbeispiel der erste dielektrische Wellenleiter 10 aus, der bevorzugt als eine Art Profilleiste oder Strangprofil ausgebildet ist. Jene Profilleiste oder jenes Strangprofil weist dabei bevorzugt einen über die gesamte Längsausdehnung des ersten dielektrischen Wellenleiters 10 hinweg einheitlichen Querschnitt auf.

Die entsprechende Profilleiste bzw. das entsprechende Strangprofil ist weiter bevorzugt einteilig, einstückig und quasi monolithisch ausgestaltet und zum Beispiel aus Teflon oder einen Polyethylen gefertigt.

Die Form des ersten dielektrischen Wellenleiters 10 lässt sich dabei bevorzugt in verschiedene Bereiche unterteilen, die sich hinsichtlich ihrer Funktion unterscheiden. So weist der erste dielektrische Wellenleiter 10 zweckdienlicherweise einen Leiterrumpf 22 auf, der beispielsweise quaderförmig ausgestaltet ist und an den sich üblicherweise eine Haltestruktur 24 anschließt. Dabei dient der Leiterrumpf 22 in erster Linie zur Durchleitung elektromagnetischer Wellen und die Haltestruktur 24 in erster Linie zur Fixierung des ersten dielektrischen Wellenleiters 10 an der Gantry-Tragstruktur 18. Das heißt, dass infolge der Ausgestaltung des ersten dielektrischen Wellenleiters 10 sich die elektromagnetischen Felder bei der Durchleitung elektromagnetischer Wellen durch den ersten dielektrischen Wellenleiter 10 vor allem auf den Leiterrumpf 22 konzentrieren und sich nur in geringem Ausmaße in die Haltestruktur 24 ausdehnen.

Eine mögliche Ausführung eines ersten dielektrischen Wellenleiters 10 ist in FIG 2 dargestellt. Hierbei weist der erste dielektrische Wellenleiter 10 im Querschnitt gesehen einem quaderförmigen Leiterrumpf 22 auf, an den sich an zwei gegenüberliegenden Seiten in Querrichtung 26 gesehen zwei Haltestege 28 anschließen, die den Leiterrumpf 22 wie zwei seitlicher Flügel flankieren und die in dieser Ausführungsvariante die Haltestruktur 24 ausbilden. Dabei bilden dann der Leiterrumpf 22 und die beiden Haltestege 28 eine Art T-Form aus. Wie aus FIG 2 weiter hervorgeht, ist der erste dielektrische Wellenleiter 10 in diesem Ausführungsbeispiel in der Nut 16 der Gantry-Tragstruktur 18 versenkt angeordnet und bei ausgebildeter Steckverbindung hintergreifen die beiden Haltestege 28 eine Hinterschneidung 30 in der Nut 16, so dass der erste dielektrische Wellenleiter 10 durch Formschluss in der Nut 16 gehalten wird.

Weiter ist der Kontakt zwischen dem ersten dielektrischen Wellenleiter 10 und der Gantry-Tragstruktur 18 im Bereich der Nut 16 auf ein Minimum beschränkt, und zwar auf den Bereich der äußeren Enden der Haltestege 28. In allen übrigen Bereichen ist um den ersten dielektrischen Wellenleiter 10 herum ein Freiraum gegeben, in dem typischerweise Luft vorliegt.

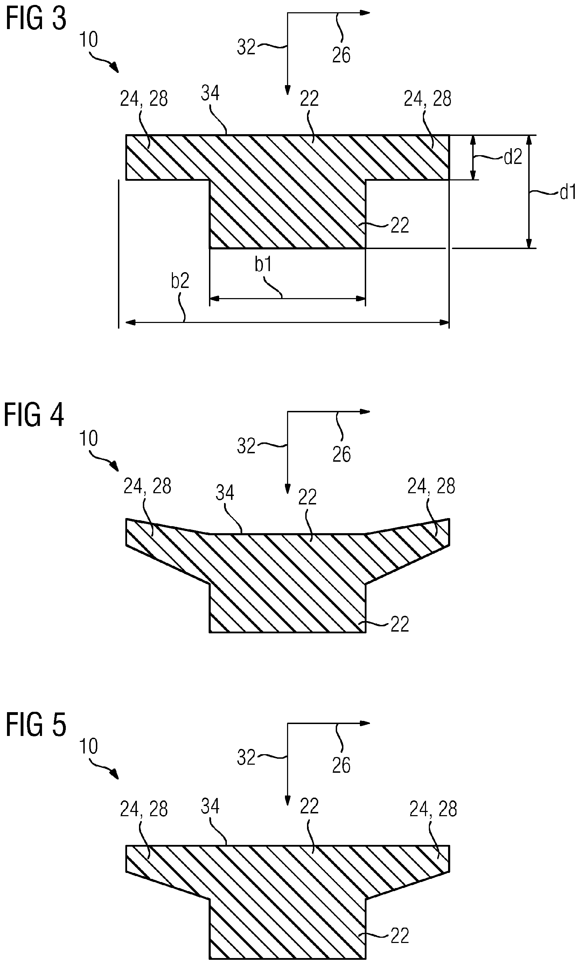

Beispielhafte Abmessungen eines für eine hier vorgestellte Bildgebungsvorrichtung geeigneten ersten dielektrischen Wellenleiters 10 sind in FIG 3 angedeutet, wobei b2=10mm, b1=4mm, d1=2mm und d2=1mm.

Weitere Ausführungsvarianten des ersten dielektrischen Wellenleiters 10 sind in FIG 4 bis FIG 7 wiedergegeben, wobei sich diese vom zuvor beschriebenen durch einen abweichenden Querschnitt unterscheiden. Gemeinsam ist allen Ausführungsvarianten ein quaderförmiger Leiterrumpf 22, an den sich je ein Haltesteg 28 an zwei gegenüberliegenden Seiten anschließt.

In FIG 4 und FIG 5 verjüngen sich die Haltestege 28 dabei in Querrichtung 26 und entgegen der Querrichtung 26, also jeweils in eine Richtung, die vom Leiterrumpf 22 weg zeigt. Im Falle der Ausführungsvariante gemäß FIG 4 sind die Haltestege 28 zudem abgewinkelt, und zwar entgegen der Steckrichtung 32 der Steckverbindung, in die der erste dielektrische Wellenleiter 10 zur Ausbildung der Steckverbindung geführt wird.

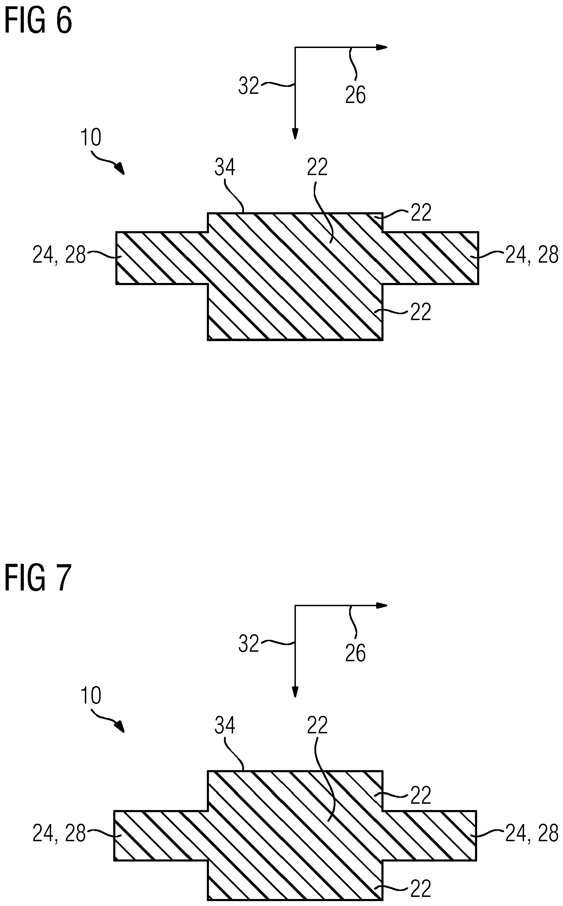

Im Gegensatz zu den Ausführungsvarianten gemäß FIG 2 bis FIG 5 sind die Haltestege 28 in den beiden Ausführungsvarianten gemäß FIG 6 und FIG 7 nicht bündig an den Leiterrumpf 22 angeformt, sondern stattdessen an einer in Steckrichtung 32 versetzten Position, also quasi etwas unterhalb der Oberseite 34 des Leiterrumpfes 22, welche im Computertomographen 2 dem zweiten dielektrischen Wellenleiter 12 zugewandt ist. Diese beiden Ausführungsvarianten sind insbesondere dann von Vorteil, wenn der erste dielektrische Wellenleiter 10 nicht Teil der rotierbaren Gantry 6, sondern der feststehenden Basis 4 ist und dementsprechend an einer Tragstruktur der feststehenden Basis 4 mittels Steckverbindung befestigt ist. In diesem Fall treten dann im Betrieb typischerweise keine größeren Kräfte auf, also insbesondere keine Fliehkräfte, die der Steckverbindung entgegenwirken, und dementsprechend ist dann eine derartige Steckverbindung für geringere Kräfte, insbesondere entgegen der Steckrichtung 32 wirkende Kräfte, ausgelegt.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den Gegenstand der Erfindung zu verlassen. Insbesondere sind ferner alle im Zusammenhang mit dem Ausführungsbeispiel beschriebenen Einzelmerkmale auch auf andere Weise miteinander kombinierbar, ohne den Gegenstand der Erfindung zu verlassen.

## Patentansprüche

1. Datenübertragungseinheit (8), aufweisend eine Empfangseinheit sowie eine relativ zur Empfangseinheit bewegbare Sendeeinheit und eingerichtet zur Übertragung von Daten von der Sendeeinheit zur Empfangseinheit über einen Koppler (14) auch während einer Relativbewegung zwischen Sendeeinheit und Empfangseinheit, wobei der Koppler (14) einen ersten dielektrischen Wellenleiter (10) aufweist,
**dadurch gekennzeichnet, dass** zum einen der erste dielektrische Wellenleiter (10) einen Leiterrumpf (22) zur Durchleitung von elektromagnetischen Wellen aus dem Frequenzbereich 40 bis 300 GHz und eine Haltestruktur (24) zur Fixierung des ersten dielektrischen Wellenleiters (10) an einer Baueinheit aufweist und dass zum anderen die Haltestruktur (24) durch eine Anzahl Haltearme ausgebildet ist, die an den Leiterrumpf (22) angeformt sind, und/oder der erste dielektrische Wellenleiter (10) eine Art Profilleiste (10) ausbildet, wobei die den ersten dielektrischen Wellenleiter (10) ausbildende Profilleiste (10) im Querschnitt gesehen eine Anzahl Haltestege (28) aufweist, die sich quer zur Längsausdehnung der Profilleiste (10) erstrecken.

2. Datenübertragungseinheit (8) nach Anspruch 1,
**dadurch gekennzeichnet, dass** der Leiterrumpf (22) und die Haltestruktur (24) aus demselben Material bestehen und dass der erste dielektrischen Wellenleiter (10) insbesondere einstückig ausgebildet ist.

3. Datenübertragungseinheit (8) nach Anspruch 1 oder 2, **dadurch gekennzeichnet, dass** die Haltestruktur (24) einen ersten Teil (24) einer Steckverbindung (16,24) ausbildet.

4. Datenübertragungseinheit (8) nach einem der Ansprüche 1 bis 3,
**dadurch gekennzeichnet, dass** der erste dielektrische Wellenleiter (10) eine Art Profilleiste (10) ausbildet mit durchgängig einheitlichem Querschnitt.

5. Datenübertragungseinheit (8) nach Anspruch 4,
**dadurch gekennzeichnet, dass** die den ersten dielektrischen Wellenleiter (10) ausbildende Profilleiste (10) im Querschnitt gesehen eine Anzahl Haltestege (28) aufweist, die sich quer zur Längsausdehnung der Profilleiste (10) erstrecken und auch quer zu einer Steckrichtung (32) einer Steckverbindung (16,24).

6. Datenübertragungseinheit (8) nach Anspruch 5,
**dadurch gekennzeichnet, dass** im Querschnitt gesehen an zwei gegenüberliegenden Seiten des Leiterrumpfes (22) je ein Haltesteg (28) unmittelbar an den Leiterrumpf (22) angeformt ist.

7. Datenübertragungseinheit (8) nach Anspruch 5 oder 6, **dadurch gekennzeichnet, dass** sich die Haltestege (28) im Querschnitt gesehen nach außen hin verjüngen.

8. Datenübertragungseinheit (8) nach einem der Ansprüche 5 bis 7,
**dadurch gekennzeichnet, dass** die Haltestege (28) im Querschnitt gesehen abgewinkelt sind, insbesondere entgegen der Steckrichtung (32) der Steckverbindung (16,24).

9. Datenübertragungseinheit (8) nach einem der Ansprüche 5 bis 8,
**dadurch gekennzeichnet, dass** sich die Haltestege (28) im Querschnitt gesehen bündig an eine Oberseite (34) des Leiterrumpfes (22) anschließen, deren Normale entgegen der Steckrichtung (32) der Steckverbindung (16,24) gerichtet ist.

10. Bildgebungsvorrichtung (2), aufweisend eine Datenübertragungseinheit (8) nach einem der vorherigen Ansprüche.

11. Bildgebungsvorrichtung (2) nach Anspruch 10,
**dadurch gekennzeichnet, dass** die Sendeeinheit Teil einer rotierbaren Gantry (6) mit einer Gantry-Tragstruktur (18) und die Empfangseinheit Teil einer feststehenden Basis (4) ist, wobei der erste dielektrische Wellenleiter (10) insbesondere die Sendeeinheit mit ausbildet und an der Gantry-Tragstruktur (18) fixiert ist.

12. Bildgebungsvorrichtung (2) nach Anspruch 10 oder 11, **dadurch gekennzeichnet, dass** der erste dielektrische Wellenleiter (10) mit Hilfe einer Steckverbindung (16,24) an der Gantry-Tragstruktur (18) fixiert ist.

13. Bildgebungsvorrichtung (2) nach Anspruch 12,
**dadurch gekennzeichnet, dass** eine Nut (16) in der Gantry-Tragstruktur (18) einen Teil der Steckverbindung (16,24) ausbildet.

## Claims

1. Data transmission unit (8), which has a receive unit as well as a transmit unit which is able to be moved relative to the receive unit, and is configured for transmission of data from the transmit unit to the receive unit via a coupler (14) even during a relative movement between transmit unit and receive unit, wherein the coupler (14) has a first dielectric waveguide (10), **characterised in that** on the one hand the first dielectric waveguide (10) has a conductor body (22) for conveying electromagnetic waves from the frequency range 40 to 300 GHz and a retaining structure (24) for fixing the first dielectric waveguide (10) to a unit and, on the other hand, the retaining structure (24) is embodied by a number of retaining arms, which are formed on the conductor body (22), and/or the first dielectric waveguide (10) forms a kind of profile strip (10), wherein the profile strip (10) embodying the first dielectric waveguide (10), seen in cross-section, has a number of retaining webs (28), which extend transversely to the longitudinal extent of the profile strip (10).

2. Data transmission unit (8) according to claim 1, **characterised in that** the conductor body (22) and the retaining structure (24) consist of the same material and that the first dielectric waveguide (10) is in particular embodied in one piece.

3. Data transmission unit (8) according to claim 1 or 2, **characterised in that** the retaining structure (24) forms a first part (24) of a plug connection (16, 24).

4. Data transmission unit (8) according to one of claims 1 to 3, **characterised in that** the first dielectric waveguide (10) forms a kind of profile strip (10) with an end-to-end uniform cross-section.

5. Data transmission unit (8) according to claim 4, **characterised in that** the profile strip (10) embodying the first dielectric waveguide (10), seen in cross-section, has a number of retaining webs (28), which extend transversely to the longitudinal extent of the profile strip (10) and also transversely to a plug-in direction (32) of a plug connection (16, 24).

6. Data transmission unit (8) according to claim 5, **characterised in that**, seen in cross-section, a retaining web (28) is formed directly on the conductor body (22) in each case on two opposing sides of the conductor body (22).

7. Data transmission unit (8) according to claim 5 or 6, **characterised in that** the retaining webs (28), seen in cross-section, taper towards the outside.

8. Data transmission unit (8) according to one of claims 5 to 7, **characterised in that** the retaining webs (28), seen in cross-section, are angled, in particular against the plug-in direction (32) of the plug connection (16, 24).

9. Data transmission unit (8) according to one of claims 5 to 8, **characterised in that** the retaining webs (28), seen in cross-section, have a flush joint to an upper side (34) of the conductor body (22), of which the normal is directed against the plug-in direction (32) of the plug connection (16, 24).

10. Imaging facility (2), having a data transmission unit (8) according to one of the preceding claims.

11. Imaging facility (2) according to claim 10,
**characterised in that** the transmit unit is part of a rotatable gantry (6) with a gantry support structure (18) and the receive unit is part of a fixed base (4), wherein the first dielectric waveguide (10) in particular forms the transmit unit as well and is fixed to the gantry support structure (18).

12. Imaging facility (2) according to claim 10 or 11, **characterised in that** the first dielectric waveguide (10) is fixed to the gantry support structure (18) with the aid of a plug connection (16, 24).

13. Imaging facility (2) according to claim 12,
**characterised in that** a groove (16) in the gantry support structure (18) forms a part of the plug connection (16, 24).

## Revendications

1. Unité (8) de transmission de données, comportant une unité de réception, ainsi qu'une unité d'émission, mobile par rapport à l'unité de réception, et conçue pour la transmission de données de l'unité d'émission à l'unité de réception par un coupleur (14) également pendant un déplacement relatif entre l'unité d'émission et l'unité de réception, le coupleur (14) ayant un premier guide d'onde (10) diélectrique, **caractérisée en ce que**, d'une part, le premier guide d'onde (10) diélectrique a un premier tronçon (22) de passage d'ondes électromagnétiques dans la plage de fréquence de 40 à 300 GHz et une structure (24) de maintien pour l'immobilisation du premier guide d'onde (10) diélectrique sur une unité de construction et **en ce que**, d'autre part, la structure (24) de maintien est constituée d'un certain nombre de bras de maintien, venus de matière avec le tronçon (22), et/ou le premier guide d'onde (10) diélectrique constitue une sorte de baguette (10) profilée, la baguette (10) profilée, constituant le premier guide d'onde (10) diélectrique, ayant, considérée en section transversale, un certain nombre de réglettes (28) de maintien, qui s'étendent transversalement à l'étendue longitudinale de la baguette (10) profilée.

2. Unité (8) de transmission de données suivant la revendication 1,
**caractérisée en ce que** le tronçon (22) et la structure (24) de maintien sont en le même matériau et **en ce que** le premier guide d'onde (10) diélectrique est constitué, notamment, d'une seule pièce.

3. Unité (8) de transmission de données suivant la revendication 1 ou 2,
**caractérisée en ce que** la structure (24) de maintien constitue une première partie (24) d'une liaison (16, 24) à enfichage.

4. Unité (8) de transmission de données suivant l'une des revendications 1 à 3,
**caractérisée en ce que** le premier guide d'onde (10) diélectrique constitue une sorte de baguette (10) profilée de section transversale uniforme continûment.

5. Unité (8) de transmission de données suivant la revendication 4,
**caractérisée en ce que** la baguette (10) profilée, constituant le premier guide d'onde (10) diélectrique, a, considérée en section transversale, un certain nombre de réglettes (28) de maintien, qui s'étendent transversalement à l'étendue longitudinale de la baguette (10) profilée et également transversalement à une direction (32) d'enfichage d'une liaison (16, 24) à enfichage.

6. Unité (8) de transmission de données suivant la revendication 5,
**caractérisée en ce que**, considérée en section transversale, viennent de matière directement sur le tronçon (22), sur deux côtés opposés du tronçon (22), respectivement, une réglette (28) de maintien.

7. Unité (8) de transmission de données suivant la revendication 5 ou 6,
**caractérisée en ce que** les réglettes (28) de maintien se rétrécissent, considérées en section transversale, vers l'extérieur.

8. Unité (8) de transmission de données suivant l'une des revendications 5 à 7,
**caractérisée en ce que** les réglettes (28) de maintien sont coudées, considérées en section transversale, notamment dans le sens contraire à la direction (32) d'enfichage de la liaison (16, 24) à enfichage.

9. Unité (8) de transmission de données suivant l'une des revendications 5 à 8,
**caractérisée en ce que** les réglettes (28) de maintien sont, considérées en section transversale, à affleurement avec un côté (34) supérieur du tronçon (22), dont la normale est dirigée en sens contraire au sens (32) d'enfichage de la liaison (16, 24) à enfichage.

10. Installation (2) d'imagerie ayant une unité (8) de transmission de données suivant l'une des revendications précédentes.

11. Installation (2) d'imagerie suivant la revendication 10, **caractérisée en ce que** l'unité d'émission fait partie d'un portique (6) tournant ayant une structure (18) porteuse de portique et l'unité de réception fait partie d'un socle (4) fixe, le premier guide d'onde (10) diélectrique constituant notamment l'unité d'émission et étant immobilisé sur la structure (18) porteuse de portique.

12. Installation (2) d'imagerie suivant la revendication 10 ou 11,
**caractérisée en ce que** le premier guide d'onde (10) diélectrique est immobilisé sur la structure (18) porteuse de portique à l'aide d'une liaison (16, 24) à enfichage.

13. Installation (2) d'imagerie suivant la revendication 12, **caractérisée en ce qu'**une rainure (16) de la structure (18) porteuse de portique constitue une partie de la liaison (16, 24) à enfichage.
